# FASCICULE DE BREVET EUROPEEN

(11) **EP 2 744 893 B1**
(45) Date de publication et mention de la délivrance du brevet: **22.03.2017**
(21) Numéro de dépôt: 12748206.5
(22) Date de dépôt: 14.08.2012
(51) Int. Cl.: C12N 5/0789

(54) **MODÉLISATION IN VITRO DES NICHES MÉDULLAIRES À CELLULES SOUCHES HÉMATOPOÏÉTIQUES : UN OUTIL POUR ÉTUDIER LA RÉGULATION DE L'HÉMATOPOÏÈSE, ÉVALUER LE POTENTIEL DE NICHAGE D'UN GREFFON HÉMATOPOIÈTIQUE ET TESTER LA PHARMACO-TOXICOLOGIE DE MÉDICAMENTS**
IN-VITRO-MODELLIERUNG VON MEDULLÄREN NESTERN HÄMATOPOIETISCHER STAMMZELLEN: WERKZEUG ZUR UNTERSUCHUNG DER REGULIERUNG DER HÄMATOPOESE, BEWERTUNG DES EINNESTUNGSPOTENZIALS EINES HÄMATOPOETISCHEN TRANSPLANTATS UND PRÜFUNG DER PHARMAKOTOXIKOLOGIE VON ARZNEIMITTELN
IN VITRO MODELLING OF HAEMATOPOIETIC STEM CELL MEDULLARY NESTS: A TOOL FOR STUDYING THE REGULATION OF HAEMATOPOIESIS, EVALUATING THE NESTING POTENTIAL OF A HAEMATOPOIETIC GRAFT AND TESTING THE PHARMACOTOXICOLOGY OF MEDICAMENTS

(30) Priorité: 16.08.2011 FR 1157358
(43) Date de publication de la demande: 25.06.2014
(73) Titulaire: Etat Francais (Ministere de La Defense) Service de Sante des Armees, 75614 Paris Cedex 12 (FR); Institut National de la Santé et de la Recherche Médicale, 75013 Paris (FR)
(72) Inventeur: LATAILLADE, Jean-Jacques, F-78990 Elancourt (FR); LE BOUSSE-KERDILES, Marie-Caroline, F-92340 Bourg-la-Reine (FR)
(74) Mandataire: Lavoix
(86) Numéro de dépôt international: PCT/EP2012/065905
(87) Numéro de publication internationale: WO 2013/024096

(56) Documents cités:
- WO-A2-99/64566
- SINGBRANT S ET AL: "Defining the hematopoietic stem cell niche: the chicken and the egg conundrum.", JOURNAL OF CELLULAR BIOCHEMISTRY, vol. 112, no. 6, juin 2011 (2011-06), pages 1486-1490, XP055023980, ISSN: 1097-4644, DOI: 10.1002/JCB.23085
- KIEL M J & MORRISON S J: "Uncertainty in the niches that maintain haematopoietic stem cells.", NATURE REVIEWS. IMMUNOLOGY, vol. 8, no. 4, avril 2008 (2008-04), pages 290-301, XP055023983, ISSN: 1474-1741, DOI: 10.1038/nri2279
- BRACCINI A ET AL: "Three-dimensional perfusion culture of human bone marrow cells and generation of osteoinductive grafts.", STEM CELLS, vol. 23, no. 8, septembre 2005 (2005-09), pages 1066-1072, XP002559990, ISSN: 1066-5099, DOI: 10.1634/stemcells.2005-0002
- NAKAMURA S ET AL: "Effect of calcium ion concentrations on osteogenic differentiation and hematopoietic stem cell niche-related protein expression in osteoblasts.", TISSUE ENGINEERING PART A, vol. 16, no. 8, août 2010 (2010-08), pages 2467-2473, XP055024003, ISSN: 1937-335X, DOI: 10.1089/ten.tea.2009.0337
- CONRAD V ET AL: "Expansion and differentiation of haemopoietic progenitor cells on endothelialized hydroxyapatite under static conditions.", BRITISH JOURNAL OF HAEMATOLOGY, vol. 105, no. 1, avril 1999 (1999-04), pages 40-49, XP002236103, ISSN: 0007-1048
- DE BARROS A P D N ET AL: "Osteoblasts and Bone Marrow Mesenchymal Stromal Cells Control Hematopoietic Stem Cell Migration and Proliferation in 3D In Vitro Model", PLOS ONE, vol. 5, no. 2, E9093, 8 février 2010 (2010-02-08), XP055023102, ISSN: 1932-6203, DOI: 10.1371/journal.pone.0009093 cité dans la demande
- CALVI L M ET AL: "Osteoblastic cells regulate the haematopoietic stem cell niche.", NATURE, vol. 425, no. 6960, 23 octobre 2003 (2003-10-23), pages 841-846, XP002389181, ISSN: 1476-4687, DOI: 10.1038/nature02040
- DE SMEDT M ET AL: "Human bone marrow CD34<+> progenitor cells mature to T cells on OP9-DL1 stromal cell line without thymus microenvironment", BLOOD CELLS, MOLECULES & DISEASES, vol. 33, no. 3, 1 novembre 2004 (2004-11-01), pages 227-232, XP004620472, ISSN: 1079-9796, DOI: 10.1016/J.BCMD.2004.08.007

## Description

La présente invention concerne un support de culture de cellules souches hématopoïétiques (CSH) et/ou progéniteurs hématopoïétiques (PH), comprenant un biomatériau calcique, des ostéoclastes, des cellules endothéliales et des cellules souches mésenchymateuses (CSM) et/ou des ostéoblastes et/ou adipocytes. La présente invention concerne également une méthode de préparation d'un tel support de culture, et une méthode de culture *in vitro* de CSH et/ou PH. L'utilisation d'un tel support de culture pour étudier les mécanismes cellulaires impliqués dans l'hématopoïèse et/ou la différenciation des CSH / PH, et/ou pour étudier l'efficacité et/ou la toxicité d'un candidat médicament est également décrite.

### CONTEXTE DE L'INVENTION

### L'hématopoïèse adulte

L'hématopoïèse est le processus physiologique qui permet la production et le renouvellement continu et régulé de l'ensemble des éléments figurés du sang à partir d'un petit contingent de cellules appelées cellules souches hématopoïétiques (CSH). Chez l'homme adulte, l'hématopoïèse siège dans la moelle osseuse au sein de localisations spécifiques (appelées niches) où elle est étroitement régulée par le biais de facteurs sécrétés et/ou d'interactions directes avec la composante cellulaire et la matrice extracellulaire qui l'entourent, lesquelles constituent le « microenvironnement ». Une dérégulation de ce processus conduit, dans de nombreux cas, au développement d'hémopathies malignes. L'étude de la régulation de l'hématopoïèse aux différents niveaux de sa différenciation (CSH, progéniteurs immatures, progéniteurs commis, cellules matures et fonctionnelles) a permis de souligner l'importance du microenvironnement et des signaux qu'il engendre. En effet, c'est le développement de systèmes de co-culture de cellules hématopoïétiques sur des cellules médullaires stromales qui, en mimant un microenvironnement, a donné accès *in vitro*, à l'étude de la myélopoïèse et de la lymphopoïèse B ainsi que des progéniteurs immatures LTC-IC (Long Term Culture-Initiating Cells). La lymphopoïèse T *in vitro* n'a, quant à elle, été possible qu'en présence d'un microenvironnement thymique ou directement en cultures organotypiques de type FTOC (Fetal Thymic Organ Culture). Son étude sur stroma médullaire a récemment été rendue possible par la modification de ces stromas (surexpression de Delta like-1 par transfert de gène) (De Smedt et al, Blood Cells Mol Dis, 2004, 33(3):227-32).

Ces données soulignent l'importance de mieux comprendre les signaux émis par le microenvironnement pour étudier et analyser l'hématopoïèse normale ou pathologique dans sa composante globale. Ceci est d'autant plus important que plusieurs études récentes réalisées chez la souris suggèrent que des perturbations du microenvironnement hématopoïétique/des niches peuvent conduire au développement de syndromes myéloprolifératifs (SMP). Chez l'homme également, un nombre croissant d'arguments est en faveur du rôle du microenvironnement dans la pathogénèse des leucémies et des SMP en procurant un environnement favorable au développement du clone pathologique, au détriment de l'hématopoïèse normale. Cette hypothèse est soutenue par l'observation du développement de SMP chez certains patients allogreffés, alors que les donneurs concernés n'en développent pas.

### Les niches médullaires à cellules souches hématopoïétiques

Bien que le concept de niche hématopoïétique ait été introduit pour la première fois en 1978 par Schofield, ce n'est que depuis le début des années 2000 qu'un faisceau d'arguments expérimentaux concoure à la démonstration de son existence.

Les connaissances actuelles du microenvironnement hématopoïétique font état de l'existence de deux niches régulant les CSH et respectivement appelées niche endostéale et niche vasculaire. La niche endostéale se situe au contact de l'os et est composée d'ostéoblastes, de fibroblastes et d'adipocytes qui ont tous pour origine la cellule souche mésenchymateuse (CSM) ainsi que d'ostéoclastes dérivant des CSH et impliqués dans la résorption osseuse. Des études très récentes montrent que les ostéoblastes et adipocytes peuvent dériver de CSM Nestine⁺, dont la quiescence, la prolifération et la différenciation seraient régulées par le système nerveux sympathique (SNS), suggérant donc le rôle du système nerveux central dans le modelage des niches, la régulation des CSH et de leur mobilisation. La niche vasculaire, quant à elle, consiste en un réseau de vaisseaux fenêtrés, constitués de cellules endothéliales dérivant des cellules souches endothéliales (CSE). Des cellules dites «CAR» (CXCL12 Abundant Reticular cells), présentes dans la moelle osseuse, participeraient également à la constitution de ces niches *via* la production de CXCL12/SDF-1.

Les CSH entretiennent un dialogue constant avec leur niche, où elles sont régulées *via* des contacts directs avec différentes cellules stromales, ainsi que par des facteurs microenvironnementaux (concentrations en Ca²⁺ et en O₂). Un certain nombre de couples ligands/récepteurs tels que Angiopoietin-1/Tie2, Jagged/Notch, Frizzled/Wnt, Sonic Hedgehog/Patched, ainsi que les molécules d'adhérence de type VLA4, VLA5, CD44, N-cadherin sont impliqués dans les interactions entre l'ostéoblaste et la CSH, et réguleraient l'équilibre quiescence/autorenouvellement de ces dernières. De plus, la fonctionnalité SP (Side Population) d'exclusion de drogues, une des caractéristiques des CSH, pourrait être intimement liée à leur localisation au sein des niches et notamment, endostéales. Les facteurs diffusibles sont quant à eux représentés par certaines hormones (hormone paratyroïdienne, sérotonine,...), des BMPs (bone morphogenic protein), des cytokines (SCF, VEGF, TPO...) et des chimiokines (CXCL12/SDF-1, IL8...).

La niche endostéale, siège d'une hypoxie relative au contact de l'os, serait impliquée dans le maintien à l'état quiescent des cellules souches, *via* des interactions avec des cellules ostéocompétentes de la lignée mésenchymateuse (ostéoblastes, adipocytes, fibroblastes) et de la lignée hématopoïétique (ostéoclastes et cellules endothéliales).

Outre son rôle structurel de support, la matrice extracellulaire (MEC) fonctionne également comme un régulateur clé de la prolifération/différenciation/survie des CSH/PH au sein des niches. Parmi les composants de cette matrice on trouve des protéines structurelles incluant les collagènes et l'élastine, des protéines spécialisées comme la fibronectine et la laminine, et des protéines régulatrices comme les métalloprotéases et les protéoglycannes. Les protéoglycannes constituent une famille de protéines dont l'intérêt majeur est porté par ses chaines de glycosaminoglycannes (GAGs). Ces GAGs interviennent dans la biodisponibilité des facteurs de croissance et des chimiokines et les protègent de leur dégradation protéolytique.

A l'heure actuelle, alors que l'impact du système osseux sur l'hématopoïèse commence à être mieux connu, peu de travaux ont étudié le rôle réciproque de l'hématopoïèse dans la physiologie du remodelage osseux. La niche vasculaire, où la concentration en oxygène est plus élevée, serait plutôt impliquée dans la prolifération et la différenciation des CSH, bien que des travaux récents aient démontré l'importance des cellules endothéliales dans l'auto-renouvellement des CSH. Cependant, à ce jour, il n'est définitivement pas établi si ces deux niches sont distinctes, ou si elles contribuent à une niche commune.

### Comprendre la régulation de l'hématopoïèse et tester la pharmaco-toxicologie de médicaments

Les mécanismes impliqués dans les interactions entre les CSH et les cellules ostéocompétentes au sein de leurs niches régulatrices demeurent encore mal connus, que ce soit en physiologie ou en pathologie. La compréhension de ces mécanismes est en effet limitée du fait de l'absence d'outil *in vitro* permettant d'étudier l'hématopoïèse dans un contexte global intégrant en particulier ses aspects microenvironnementaux.

D'autre part, le développement de nouvelles molécules thérapeutiques nécessite la mise au point de tests appropriés pour évaluer leur toxicité et leur efficacité sur l'hématopoïèse et potentiellement sur le remodelage osseux. Un tel outil présente donc un intérêt majeur sur les plans cognitif, pharmacologique et thérapeutique, en particulier en hématologie (di Maggio et al, Biomaterials, 2011, 32(2):321-9). En effet, actuellement, il n'existe pas, ou très peu, de système de culture permettant l'étude de l'hématopoïèse normale ou pathologique dans le contexte de son microenvironnement : la composante « niche » est, soit absente (culture liquide en condition de cytokines), soit restreinte et mal adaptée (co-culture sur lignées stromales), soit non accessible (expérimentation *in vivo*, greffe). Parmi les rares études rapportées à ce jour, les modèles développés sont limités à des interactions entre certaines cellules stromales et les CSH, et ils ne prennent pas en compte la niche dans sa globalité (de Barros et al, PLoS One, 2010, 8;5(2)).

Il existe donc un intérêt majeur à modéliser une niche en 2 (2D) ou 3 dimensions (3D) pour disposer d'un outil *in vitro* accessible et modulable pour l'étude de l'hématopoïèse dans un contexte le plus proche de la physiologie.

L'objet de la présente demande est donc de proposer un modèle de niche hématopoïétique en 2D ou 3D, recréant la complexité de l'environnement médullaire.

L'invention consiste à cellulariser des supports biologiques (fragments osseux décellularisés) ou manufacturés, de richesse calcique variable, avec différents types de cellules stromales (ostéoblastes, ostéoclastes, adipocytes, cellules mesenchymateuses, cellules endothélailes..) et à co-cultiver, sur ces supports cellularisés, des cellules souches ou des progéniteurs hématopoïétiques (SP, ALDH^{fortes}, CD34⁺, Lin⁻...). Des constituants de la matrice extracellulaire (GAG, fibronectine, collagènes...) sont greffés sur ces supports afin d'assurer une bonne biodisponibilité des facteurs produits par les cellules stromales. Afin de reproduire au plus près les variations de concentration en oxygène du microenvironnement médullaire, les co-cultures sont réalisées à des concentrations d'O₂ variant de 0,1% à 20%, préférentiellement de 1% à 3%.

Ce modèle permet de révéler le potentiel SP des cellules souches et pourrait constituer un test d'évaluation *in vitro* du potentiel de nichage d'un greffon hématopoïètique. Il permet également d'étudier le rôle des éléments cellulaires et humoraux constituant la niche hématopoïétique sur la mise en cycle, la prolifération, la différenciation et la mobilisation des CSH. Ce système permettra également d'étudier le rôle des cellules hématopoïétiques sur les cellules ostéocompétentes et le remodelage osseux. Enfin, ce modèle peut être utilisé comme outil pharmacologique et toxicologique pour tester *in vitro* de nouvelles drogues ciblant les maladies hématologiques et pouvant toucher les éléments cellulaires constituant la niche hématopoïétique. Ce modèle est donc utilisable pour des études pharmacologiques et toxicologiques de médicaments. Cet outil permet également d'étudier le rôle des niches hématopoïétiques dans la dérégulation de l'hématopoïèse caractérisant certaines hémopathies malignes.

### DESCRIPTION DE L'INVENTION

### Support de culture

Un premier aspect de l'invention correspond à un support de culture de cellules souches hématopoïétiques (CSH) et/ou progéniteurs hématopoïétiques (PH), comprenant :
a. un biomatériau calcique ;
b. des ostéoclastes ;
c. des cellules endothéliales ; et
d. des cellules souches mésenchymateuses (CSM) et/ou des ostéoblastes et/ou adipocytes.

Le support de culture de cellules souches hématopoïétiques (CSH) et/ou progéniteurs hématopoïétiques (PH) selon l'invention comprend une composante cellulaire et une composante acellulaire, nommée « biomatériau calcique », qui joue le rôle de support ostéoconducteur. Le biomatériau calcique peut être simple ou multiple, de sorte que les cellules constituant le support de culture et les cellules en culture peuvent être ensemencées sur un même biomatériau ou sur des biomatériaux différents (de natures identiques ou différentes), par exemple un par type cellulaire. Par « biomatériaux différents » on entend des entités individualisées provenant d'un même biomatériau, ou des biomatériaux ayant des compositions différentes.

La composition du biomatériau calcique peut varier. Le biomatériau calcique peut comprendre ou être constitué d'hydroxyapatite (HA) et de phosphate tricalcique (TCP). Par exemple, le biomatériau calcique peut correspondre à des fragments osseux décellularisés ou à des matériaux synthétiques. Les proportions d'HA et de TCP peuvent varier. Le fait de faire varier les proportions de ces deux constituants peut par exemple permettre d'obtenir des biomatériaux présentant des porosités différentes. En particulier, la proportion en HA du biomatériau peut varier entre 55% et 75%, de préférence entre 60% et 70%. La proportion en TCP du biomatériau peut varier entre 25% et 45%, de préférence entre 30% et 40%.

Le biomatériau calcique est bidimensionnel ou tridimensionnel. L'utilisation d'un biomatériau calcique de type tridimensionnel peut en particulier permettre de réaliser la culture des CSH et/ou des PH dans ces conditions physiologiques plus proches de celles présentes au sein de la niche hématopoïétique naturelle.

Selon un mode de réalisation spécifique, le biomatériau calcique est sélectionné dans le groupe constitué des biomatériaux de type B2D ou de type B3D (BD Biocoat™ Osteologic™ Bone Cell Culture System) et du Calciresorb 35® (Ceraver, France). Les biomatériaux de B2D ou B3D sont des matériaux synthétiques multi-phasés à base de phosphate de calcium qui présentent une excellente biocompatibilité et sont adaptés pour permettre la prolifération et la différenciation de cellules de nombreux types. Le biomatériau B3D présente une structure réticulée à pores ouverts et possède une taille de pores et une porosité idéales pour des études *in vitro*. Le Calciresorb 35® est un mélange de phosphate tricalcique β Ca₃(PO₄)₂ et d'hydroxyapatite Ca₁₀(PO₄)₆(OH)₂ synthétiques sous forme de blocs poreux. Cette céramique biphasée est composée de 65% de HA et de 35% de βTCP. Sa porosité est d'environ 45 %, la porosité totale étant constituée d'une macroporosité (pores de 100 à 400 µm) et d'une microporosité (pores inférieurs à 10 µm).

Les cellules constituant la composante cellulaire du support de culture selon l'invention peuvent avoir différentes origines. En particulier, elles peuvent provenir de moelle osseuse humaine. Les cellules mononucléées de moelle osseuse peuvent par exemple être isolées à partir de fragments d'os spongieux issus de résidus opératoires de patients opérés pour prothèse totale de hanche. Les cellules endothéliales comprises dans le support de culture selon l'invention peuvent également provenir de cellules isolées à partir du sang de patient, telles que les progéniteurs endothéliaux circulant du sang (PEC) présents dans le sang périphérique ou les cellules hématopoïétiques mononucléées sanguines.

Les ostéoclastes peuvent être obtenus à partir de progéniteurs hématopoïétiques par différenciation spécifique. Le phénotype ostéoclastique peut par exemple être validé par coloration de May Grumwald Giemsa (MGG) permettant de mettre en évidence l'aspect multinucléé résultant de la fusion des cellules ostéoclastiques, et par la mise en évidence de l'activité phosphatase acide tartrate résistante (TRAP).

Les cellules endothéliales comprises dans le support de culture selon l'invention sont des cellules hématopoïétiques non engagées (dites « lineage negative » ou Lin-). De plus, elles expriment les molécules CD144 et KDR à leur surface. Les cellules endothéliales de l'invention peuvent par exemple être obtenues à partir de cellules mononuclées de la moelle osseuse, en effectuant une déplétion immunomagnétique des cellules « lineage positives » (permettant d'éliminer la population majoritaire des cellules hématopoïétiques engagées), puis un tri CD144/KDR. Les cellules triées peuvent ensuite être cultivées en milieu endothélial. Le milieu de culture endothélial peut par exemple être le milieu de type EMG2-MV commercialisé par Lonza ou un milieu équivalent. Les progéniteurs endothéliaux peuvent également être obtenus à partir du sang périphérique (PEC). Pour cela, les colonies dérivées des PEC sont générées par adhérence au plastique à partir des cellules mononucléées obtenues à partir de 20 mL de sang.

Le support de culture selon l'invention comprend par ailleurs des CSM et/ou des ostéoblastes et/ou des adipocytes. En particulier, le support de culture selon l'invention peut comprendre des CSM ou des ostéoblastes ou des adipocytes.

Les CSM peuvent provenir de moelle osseuse humaine et être isolées par adhérence au plastique. Par exemple, les CSM peuvent être obtenues par culture de cellules mononucléées de moelle osseuse isolées à partir de fragments d'os spongieux issus de résidus opératoires de patients opérés pour prothèse totale de hanche. Dans le cas où le support de culture comprend des CSM, celles-ci sont ensuite différenciées en ostéoblastes et/ou adipocytes, comme décrit ci-après pour la méthode de préparation du support de culture. Elles sont caractérisées phénotypiquement par la co-expression d'un certain nombre de marqueurs tels que par exemple CD73, CD90, CD105, CD146, et l'absence d'expression d'autres marqueurs, en particulier le CD45 et le CD34.

Le support de culture selon l'invention peut comprendre des ostéoblastes, lesquels peuvent par exemple provenir d'une différenciation spécifique des CSM. Le phénotype ostéoblastique peut être évalué au microscope à contraste de phase par appréciation du niveau de minéralisation, par immunohistochimie par mise en évidence de l'activité phosphatase alcaline (PAL) grâce à une réaction chimique au naphtol AS-BIphosphate, et par immunofluorescence indirecte (IFI) avec mise en évidence de l'ostéocalcine, de l'ostéopontine et de la PAL.

Le support de culture selon l'invention peut comprendre des adipocytes, lesquels peuvent par exemple provenir d'une différenciation spécifique des CSM. La nature adipocytaire des cellules peut être vérifiée par l'examen microscopique à contraste de phase montrant la présence de vacuoles lipidiques, et par une coloration immunohistochimique à l'oil red O fixant les vacuoles lipidiques.

Selon un mode de réalisation particulier, le support de culture selon l'invention comprend en outre un ou des composants de la matrice extracellulaire (MEC).

L'expression « composants de la matrice extracellulaire » désigne tout composé entrant dans la composition de la matrice extracellulaire. Les composés constituant la matrice extracellulaire sont bien connus de l'homme du métier et incluent, par exemple, les glycosaminoglycanes (GAG) naturels, la fibronectine, les collagènes, l'acide hyaluronique, laminine et élastine. Les composants de la matrice extracellulaire selon l'invention peuvent également correspondre à des mimétiques des GAG. En effet, ceux-ci présentent l'avantage de ne pas être dégradés par des glycanases. Selon un mode de réalisation spécifique de l'invention, les composants de la matrice extracellulaire sont des GAG naturels, des mimétiques de GAG, de la fibronectine, des collagènes, et/ou de l'acide hyaluronique. De préférence, les mimétiques de GAG utilisés sont ceux commercialisés par la société OTR3 (Organ, Tissue, Regeneration, Repair and Replacement) sous l'appellation de RGTAs pour « ReGeneraTing Agents ». Lorsqu'ils sont associés au support de culture selon l'invention, les composants de la matrice extracellulaire sont préférentiellement utilisés à une concentration de 80 à 100 ng/ml.

Selon un mode de réalisation particulier, le support de culture selon l'invention comprend en outre une colle biologique.

L'expression « colle biologique » désigne tout composé d'origine biologique permettant de maintenir les cellules entre elles et au contact des biomatériaux. Les composés pouvant être utilisés comme colle biologique sont connus de l'homme du métier. La colle biologique peut, par exemple, être obtenue à partir de plasma (colle biologique de type colle de fibrine) ou de plasma riche en plaquettes (colle biologique de type gel de plaquettes).

### Méthode de préparation d'un support de culture

Un autre aspect de l'invention concerne une méthode de préparation d'un support de culture selon l'invention, comprenant les étapes consistant à greffer un biomatériau calcique avec des ostéoclastes, des cellules endothéliales et des cellules souches mésenchymateuses (CSM) et/ou des ostéoblastes et/ou adipocytes, et optionnellement un ou des composants de la MEC.

La préparation d'un support de culture selon l'invention nécessite la mise en commun des différents stromas dans un même puits. Dans un premier temps, les ostéoclastes, les cellules endothéliales et les CSM et/ou des ostéoblastes et/ou adipocytes constituant la niche peuvent être ensemencées sur un ou plusieurs biomatériaux, 2D ou 3D. Si des CSM sont employées, celles-ci pourront par la suite être différenciées en ostéoblastes et/ou adipocytes. On obtient ainsi des BM hybrides cellularisés par les différents types de cellules stromales. Ces derniers peuvent ensuite être rassemblés dans un puits unique contenant l'ensemble des BM hybrides, pour constituer le support de culture selon l'invention. Alternativement, des cultures de chaque type cellulaire différencié peuvent être réalisées sous forme de pelotes, c'est-à-dire en les empêchant d'adhérer au support lors de leur différenciation. Les différents types cellulaires peuvent ensuite être mixés et ensemencés sur un seul et même biomatériau, comme décrit par de Barros et al (PLoS One, 2010, 8;5(2)).

Des CSH/PH pourront par la suite être ajoutées sur le support de culture. La concentration de ces CSH/PH peut varier. De manière préférentielle, la concentration en CSH/PH est comprise entre 5.10⁴ et 2.10⁵ cellules par ml et par puits, de manière encore plus préférentielle, la concentration en CSH/PH est comprise entre 10⁵ et 2.10⁵ cellules par ml et par puits.

Les ostéoclastes peuvent être obtenus à partir de progéniteurs hématopoïétiques par différenciation spécifique. En général, la différenciation des progéniteurs hématopoïétiques débute par une déplétion Lin⁻ effectuée sur colonne d'affinité afin d'éliminer les cellules différenciées exprimant les antigènes de lignée suivants : CD2, CD3, CD11b, CD14, CD15, CD16, CD19, CD56, CD123, GPA. Les cellules Lin⁻ ainsi obtenues peuvent ensuite être mises en culture sur des biomatériaux en présence d'un cocktail de cytokines comprenant par exemple SCF, Flt3L et TPO à une concentration variant de 10 à 50 ng/ml. Après 5 jours de culture, le milieu de culture peut être remplacé par un milieu de différenciation myéloïde contenant les cytokines suivantes : SCF, Flt3L, TPO, IL-6 et GM-CSF à une concentration variant de 10 à 50 ng/ml. Après 15 jours de culture, les cellules peuvent être incubées dans un milieu d'induction ostéoclastique contenant du M-CSF, RANK-L et IL-6 à une concentration variant de 10 à 20 ng/ml. La qualité de la différenciation peut être contrôlée sur un puits ne contenant pas de biomatériau. Le phénotype ostéoclastique peut par exemple être vérifié par coloration de May Grumwald Giemsa (MGG) permettant de mettre en évidence l'aspect multinucléé, résultant de la fusion des cellules ostéoclastiques, et par la mise en évidence de l'activité phosphatase acide tartrate résistante (TRAP).

Lorsque des CSM sont greffées sur le support de culture, ces cellules sont ensuite différenciées en ostéoblastes ou en adipocytes. Pour cela, les CSM peuvent être réparties en deux lots, puis chacun des lots est cultivé en présence d'un ensemble de facteurs de croissance et/ou de cytokines spécifiques, afin d'induire la différenciation des CSM d'un lot en ostéoblastes, et la différenciation des CSM de l'autre lot en adipocytes.

L'induction de la différenciation des ostéoblastes à partir des CSM peut être réalisée par l'ajout dans le milieu de culture de dexamethasone, d'acide L-ascorbique 2-phosphate et de β-glycerophosphate. La différenciation peut être observée après trois semaines de culture dans ce milieu d'induction. Ces molécules d'induction peuvent être utilisées à des concentrations comprises entre 0,05 µM et 0,2 µM pour le dexamethasone, entre 0,04 mM et 0,06 mM pour l'acide L-ascorbique 2-phosphate et entre 8 mM et 12 mM pour le β-glycerophosphate. Les concentrations préférées pour ces molécules d'induction sont les suivantes : 0,1 µM pour le dexamethasone, 0,05 mM pour l'acide L-ascorbique 2-phosphate et 10 mM pour le β-glycerophosphate.

La qualité de la différenciation des ostéoblastes peut ensuite être évaluée par différentes techniques bien connues de l'homme du métier. Par exemple, la qualité de la différenciation peut être évaluée au microscope à contraste de phase par appréciation du niveau de minéralisation, par immunohistochimie par mise en évidence de l'activité phosphatase alcaline (PAL) grâce à une réaction chimique au naphtol AS-BIphosphate, et par immunofluorescence indirecte (IFI) avec mise en évidence de l'ostéocalcine, de l'ostéopontine et de la PAL. En particulier, les réactions d'IFI peuvent être réalisées après fixation au paraformaldéhyde à 4% suivie d'une saturation/perméabilisation par du PBS enrichi par 3% d'albumine bovine et 0,1% de triton X100. Les anticorps anti-ostéocalcine, anti-ostéopontine et anti-PAL peuvent être incubés dans une solution de PBS enrichi par 1% d'albumine bovine et 0,05% de tween 20, puis les marquages sont révélés par un anticorps ciblant les immunoglobulines murines couplé à la phycoérythrine. Enfin, une contre coloration au Hoechst permettant de visualiser les noyaux peut être réalisée.

La différenciation des adipocytes peut être induite à partir de CSM par traitement avec du dexamethasone, du 3-isobutyl-1-methylxanthine (IBMX), de l'indométhacine et de l'insuline pendant neuf jours. Ces molécules peuvent être utilisés à des concentrations comprises entre 0,05 µM et 0,2 µM pour le dexamethasone, entre 0,4 mM et 0,6 mM pour le 3-isobutyl-1-methylxanthine (IBMX), entre 0,1 mM et 0,3 mM pour l'indométhacine et entre 0,005 mg/ml et 0,02 mg/ml pour l'insuline. Les concentrations préférées pour ces molécules sont les suivantes : 1 µM pour le dexamethasone, 0,5 mM pour le 3-isobutyl-1-methylxanthine (IBMX), 0,2 mM pour l'indomethacine et 0,01 mg/ml pour l'insuline.

La nature adipocytaire des cellules peut ensuite être confirmée par l'examen microscopique à contraste de phase montrant la présence de vacuoles lipidiques, et par une coloration immunohistochimique à l'oil red O fixant les vacuoles lipidiques.

Selon un mode de réalisation spécifique, les ostéoblastes et les adipocytes différenciés sont greffés sur deux supports de culture distincts ou sur un même support de culture.

La culture des différents types cellulaires constituant le support de l'invention peut être réalisée soit en normoxie, c'est-à-dire à une concentration relative en oxygène de 20%, soit en condition hypoxique plus proche de la situation physiologique dans la moelle osseuse, c'est-à-dire à des concentrations en oxygène comprises entre 1 et 5%, de préférence 3%. Selon un mode de réalisation spécifique, la préparation du support de culture est réalisée dans des conditions telles que la teneur en oxygène est comprise entre 1 et 5%. Par exemple, la préparation du support de culture peut être réalisée dans une chambre ou dans un incubateur, dans lequel la teneur en oxygène peut être mesurée au moyen d'une sonde et automatiquement ajustée à une valeur déterminée.

### Méthode de culture in vitro de CSH et/ou de PH

Un autre aspect de l'invention concerne une méthode de culture *in vitro* de cellules souches hématopoïétiques (CSH) et/ou progéniteurs hématopoïétiques (PH), comprenant les étapes consistant à :
a. ensemencer au moins un support de culture tel que défini ci-dessus, avec des cellules souches hématopoïétiques (CSH) et/ou progéniteurs hématopoïétiques (PH) ; et
b. cultiver lesdites cellules souches hématopoïétiques (CSH) et/ou progéniteurs hématopoïétiques.

Les CSH/PH peuvent être obtenus à partir de cellules hématopoïétiques mononucléées médullaires ou sanguines. Les cellules n'exprimant pas d'antigènes de différenciation tels que, par exemple, CD2, CD3, CD11b, CD14, CD15, CD16, CD19, CD56, CD123, GPA (cellules dites « lineage negatives » ou « Lin- ») peuvent être isolées par tri après marquage avec un cocktail d'anticorps ciblés contre différents antigènes de différenciation tel que, par exemple, le « Lineage cell depletion kit », commercialisé par Miltenyi Biotec. Le tri peut en particulier être réalisé par déplétion sur un automate.

Les inventeurs ont montré que la culture *in vitro* de CSH/PH sur un support de culture tel que défini dans l'invention favorise l'expansion des cellules CD34+ à partir d'une population de cellules Lin⁻ de sang périphérique.

De plus, les inventeurs ont montré que la culture *in vitro* de CSH/PH sur un support de culture tel que défini dans l'invention favorise le maintien ou l'acquisition de la fonctionnalité SP. L'expression « fonctionnalité SP » désigne la capacité des cellules souches, et en particulier des CSH, d'effluer le colorant fluorescent Hoechst-33342 ou d'autres colorants ou drogues. Cette fonctionnalité peut par exemple être évaluée par cytométrie en flux, par quantification de la fluorescence résiduelle des cellules sous la forme d'un cytogramme caractéristique, les cellules ayant la plus forte capacité d'efflux étant considérées comme les plus primitives (Goodell et al, Methods Mol Biol, 2005, 290:343-52).

Aussi, dans un mode de réalisation spécifique, les CSH/PH cultivées sur le support de culture selon l'invention n'expriment pas d'antigènes de différenciation (Lin-), expriment un haut niveau de fluorescence correspondant à un haut niveau d'activité enzymatique ALDH (ALDH^{fortes}), expriment à leur surface le CD34 (CD34+) et présentent la fonction SP. L'identification ou sélection de cellules Lin-, ALDH^{fortes} ou CD34+ peut être effectué comme détaillé dans l'Exemple 1.

Brièvement la fonction SP peut être déterminée en incubant les cellules Lin- à en présence de Hoechst-33342, par exemple à 1-10 µg/ml dans du DMEM + 2% de sérum de veau foetal, à raison de 0,25.10⁶ à 2.10⁶ cellules/ml. L'incubation est préférentiellement réalisée à 37°C pendant 60 à 120 min, en particulier pendant 90 min. Les cellules sont ensuite centrifugées à 4°C et le culot cellulaire est re-suspendu dans du HBSS sans Ca2+, ni Mg2+, à froid, à une concentration de 2 à 4.10⁶ cellules/ml. L'efflux du Hoechst-33342 peut alors être mesuré par cytométrie en flux.

La détermination de l'activité ALDH peut être effectuée en utilisant la technologie ALDEFLUOR qui utilise le substrat Bodipy™-AminoAcétAldéhyde Diéthyl Acétal (BAAA-DA) de l'enzyme ALDH-A1. Ce substrat est dissous dans du DMSO et exposé à l'action de l'HCL afin d'être converti en BAAA, substrat fluorescent de l'enzyme. Les cellules sont incubées en présence de BAAA, par exemple à une concentration de 1,5 µM à 37°C, qui est convertit par l'ALDH en un produit fluorescent (BAA) retenu dans les cellules. Dans le tampon d'incubation ALDEFLUOR, les cellules qui expriment un haut niveau d'ALDH sont celles qui présentent un haut niveau de fluorescence, comme mesuré dans le canal FL1 (fluorescence FITC) en cytométrie de flux.

Les cellules CD34⁺ peuvent être purifiées à partir de cellules mononucléées issues d'un échantillon de moelle allogénique par séparation selon un gradient de Ficoll puis reprise du culot en tampon PBS enrichi par 2% d'Albumine humaine (Vialebex, LFB) et 0,5% d'immunoglobulines polyvalentes humaines (Tégéline, LFB) et incubation au moins 5 minutes. Les cellules CD34⁺ peuvent ensuite être purifiées par n'importe quelle méthode appropriée, par exemple par incubation en présence d'un anticorps anti-CD34 couplé à des billes magnétiques et séparation sur colonne immunomagnétique.

La culture *in vitro* de CSH/PH sur le support de culture est réalisée de préférence dans un milieu de culture approprié. Les milieux adaptés à la culture des cellules *in vitro* sont bien connus de l'homme du métier. Il peut en particulier s'agir de milieux synthétiques définis additionnés de sérum ou non. Le milieu de culture approprié pour la culture *in vitro* de CSH/PH peut par exemple correspondre à du SynH (AbcellBio) ou du STEM ALPHA.A (Stem Alpha). En général, la culture est réalisée jusqu'à ce que les cellules cultivées atteignent le niveau de différenciation souhaité. Le niveau de différenciation des cellules peut être évalué en fonction du phénotype des cellules. Les cellules peuvent par exemple être cultivées jusqu'à présenter un phénotype primitif tel qu'elles n'expriment pas d'antigènes de différenciation (Lin-), qu'elles expriment un haut niveau d'enzyme ALDH (ALDH^{fortes}), qu'elles expriment à leur surface le CD34 (CD34+) et qu'elles présentent la fonction SP.

Dans un mode de réalisation particulier, la culture *in vitro* de CSH/PH sur le support de culture est réalisée en présence d'un composé candidat. En particulier, le composé candidat peut être un candidat médicament, permettant par exemple de prévenir ou de traiter des pathologies liées à une dérégulation de l'hématopoïèse, telles que certaines hémopathies malignes. La présence du composé candidat dans le milieu de culture peut permettre la réalisation d'études de pharmacologie ou de toxicologie sur ce composé. L'efficacité du composé peut par exemple être testée en comparant les phénotypes des cellules présentes dans la culture, suite à une culture en présence ou en l'absence du composé candidat. La toxicité du composé peut par exemple être testée en évaluant la quantité ou la proportion de cellules apoptotiques ou nécrotiques présentes dans la culture, suite à une culture en présence ou en l'absence du composé candidat.

### Utilisation du support de culture

Les mécanismes impliqués dans les interactions entre les CSH et les cellules ostéocompétentes au sein de leurs niches régulatrices demeurent encore mal connus, que ce soit en physiologie ou en pathologie. La compréhension de ces mécanismes est en effet limitée du fait de l'absence d'outil *in vitro* permettant d'étudier l'hématopoïèse dans un contexte global intégrant en particulier ses aspects microenvironnementaux.

Le support de culture de l'invention peut permettre d'étudier le rôle des éléments cellulaires et humoraux constituant la niche hématopoïétique sur la mise en cycle, la prolifération, la différenciation et la mobilisation des CSH. Le support de culture peut également permettre d'étudier le rôle des cellules hématopoïétiques sur les cellules ostéocompétentes et le remodelage osseux.

Aussi, un autre aspect de l'invention porte sur l'utilisation du support de culture selon l'invention pour étudier les mécanismes cellulaires impliqués dans l'hématopoïèse et/ou la différenciation des CSH / PH.

Le support de culture de l'invention peut permettre de révéler le potentiel SP des cellules souches. Ainsi, il peut permettre d'évaluer *in vitro* le potentiel de nichage d'un greffon hématopoïétique.

Aussi, un autre aspect de l'invention porte sur l'utilisation du support de culture selon l'invention pour évaluer le potentiel de nichage d'un greffon hématopoïétique.

Jusqu'à présent, il n'existait pas de système de culture adapté permettant l'étude de l'hématopoïèse normale ou pathologique dans le contexte de son microenvironnement. Le support de culture selon l'invention peut être utilisé comme un outil *in vitro* accessible et modulable pour l'étude de l'hématopoïèse dans un contexte le plus proche de la physiologie. Un tel outil présente un intérêt majeur sur les plans pharmacologique et thérapeutique, en particulier en hématologie. En effet, le développement de nouvelles molécules thérapeutiques nécessite la mise au point de tests appropriés pour évaluer leur toxicité et leur efficacité sur l'hématopoïèse et potentiellement sur le remodelage osseux. Le support de culture selon l'invention peut avantageusement être utilisé comme outil pour des études pharmacologiques et toxicologiques de médicaments, notamment pour tester *in vitro* de nouvelles drogues ciblant les maladies hématologiques et pouvant toucher les éléments cellulaires constituant la niche hématopoïétique. Enfin, le support de culture selon l'invention peut également permettre d'étudier le rôle des niches hématopoïétiques dans la dérégulation de l'hématopoïèse caractérisant certaines hémopathies malignes.

Aussi, un autre aspect de l'invention porte sur l'utilisation du support de culture selon l'invention pour étudier l'efficacité et/ou la toxicité d'un candidat médicament.

### Kit

Un dernier aspect de l'invention concerne un kit comprenant :
a. un biomatériau calcique ;
b. des ostéoclastes, ou un ensemble de cytokines et/ou facteurs de croissance permettant la différenciation de CSH en ostéoclastes;
c. des cellules endothéliales, ou un ensemble de cytokines et/ou facteurs de croissance permettant la différenciation de progéniteurs endothéliaux circulants ou de cellules mononuclées de la moelle osseuse en cellules endothéliales ;
d. des cellules souches mésenchymateuses (CSM) et/ou des ostéoblastes et/ou des adipocytes et éventuellement un ensemble de cytokines et/ou facteurs de croissance permettant la différenciation des CSM en ostéoblastes et/ou adipocytes ; et/ou la différenciation des ostéoblastes et/ou des adipocytes ; et
e. éventuellement un ou des composants de la matrice extracellulaire (MEC)

Les cytokines et/ou facteurs de croissance permettant la différenciation de CSH en ostéoclastes et compris dans le kit selon l'invention peuvent, par exemple, inclure les cytokines/facteurs de croissance suivants : SCF, Flt3, TPO, IL-6 et GM-CSF, M-CSF, et/ou RANK-L. Les concentrations préférées pour ces facteurs de croissance sont les suivantes : 10 ng/ml pour SCF, Flt3, TPO, IL-6 et GM-CSF, et 20 ng/ml pour M-CSF, RANK-L et IL-6.

Les cytokines et/ou facteurs de croissance permettant la différenciation de progéniteurs endothéliaux circulants ou de cellules mononuclées de la moelle osseuse en cellules endothéliales et compris dans le kit selon l'invention peuvent, par exemple, inclure VEGF, bFGF, EGF et IGF-1.

Les molécules permettant la différenciation des CSM en ostéoblastes et/ou la différenciation des ostéoblastes compris dans le kit selon l'invention peuvent, par exemple, inclure le dexamethasone, l'acide L-ascorbique 2-phosphate et le β-glycerophosphate. Ces molécules peuvent être utilisés à des concentrations comprises entre 0,05 µM et 0,2 µM pour le dexamethasone, entre 0,04 mM et 0,06 mM pour l'acide L-ascorbique 2-phosphate et entre 8 mM et 12 mM pour le β-glycerophosphate. Les concentrations préférées pour ces facteurs de croissance sont les suivantes : 0,1 µM pour le dexamethasone, 0,05 mM pour l'acide L-ascorbique 2-phosphate et 10 mM pour le β-glycerophosphate.

Les molécules permettant la différenciation des CSM en adipocytes et/ou la différenciation des adipocytes compris dans le kit selon l'invention peuvent, par exemple, inclure du dexamethasone, du 3-isobutyl-1-methylxanthine (IBMX), de l'indométhacine et de l'insuline. Ces molécules peuvent être utilisées à des concentrations comprises entre 0,05 µM et 0,2 µM pour le dexamethasone, entre 0,4 mM et 0,6 mM pour le 3-isobutyl-1-methylxanthine (IBMX), entre 0,1 mM et 0,3 mM pour l'indométhacine et entre 0,005 mg/ml et 0,02 mg/ml pour l'insuline. Les concentrations préférées pour ces facteurs de croissance sont les suivantes : 1 µM pour le dexamethasone, 0,5 mM pour le 3-isobutyl-1-methylxanthine (IBMX), 0,2 mM pour l'indomethacine et 0,01 mg/ml pour l'insuline.

L'invention sera décrite plus en détail dans les figures et exemples suivants.

### BRÈVE DESCRIPTION DES FIGURES

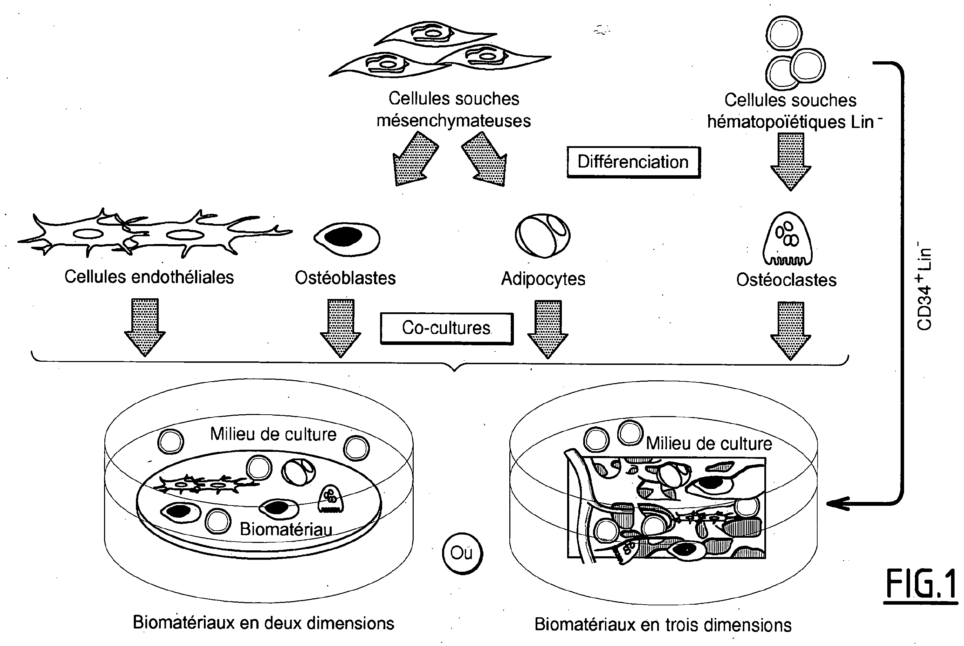

**Figure 1****. Protocole de préparation d'un support de culture 2D ou 3D selon l'invention**
   La préparation d'un support de culture selon l'invention nécessite la mise en commun des différents stromas dans un même puits. Dans un premier temps, les ostéoclastes, les cellules endothéliales et les CSM constituant la niche peuvent être ensemencées sur un ou plusieurs biomatériaux, 2D ou 3D. Les CSM peuvent par la suite être différenciées en ostéoblastes et/ou adipocytes. On obtient ainsi des BM hybrides cellularisés par les différents types de cellules stromales. Ces derniers peuvent ensuite être rassemblés dans un puits unique contenant l'ensemble des BM hybrides, pour constituer le support de culture selon l'invention.

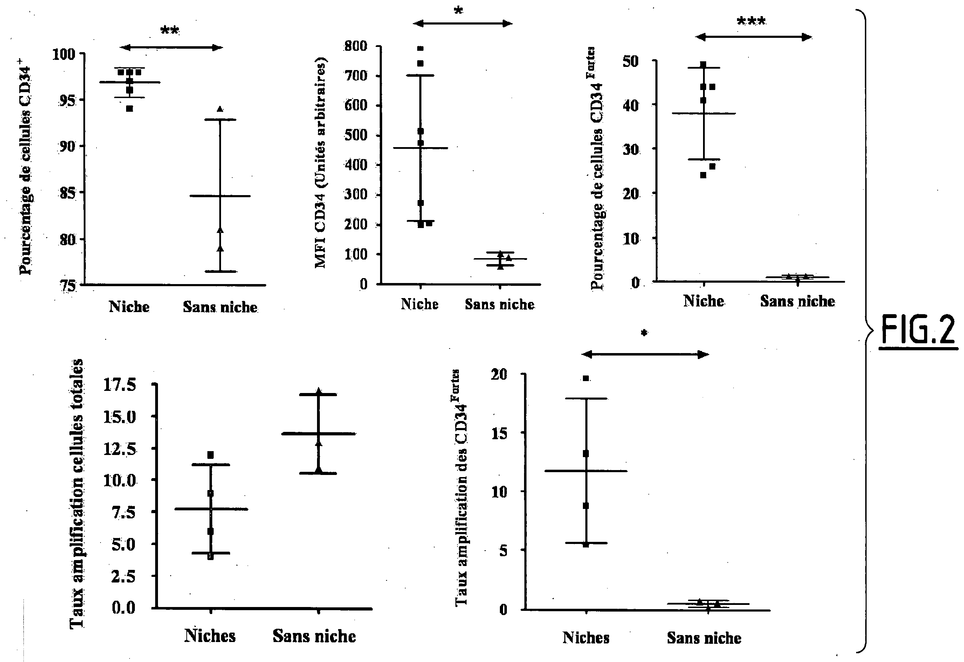

**Figure 2****. Expansion des cellules CD34⁺ sur un support de culture 2D selon l'invention (* = p<0.05 ; ** = p<0.01)**
   Une population de cellules Lin⁻ de sang périphérique de sujets sains non mobilisés, présentant une richesse initiale en cellules CD34⁺ équivalente à 30%, a été cultivé pendant 7 jours sur un support de culture en 2D selon l'invention. A l'issu de cette culture, la population CD34⁺ représente environ 96,25% +/- 1,71% (n=4) des cellules viables produites.

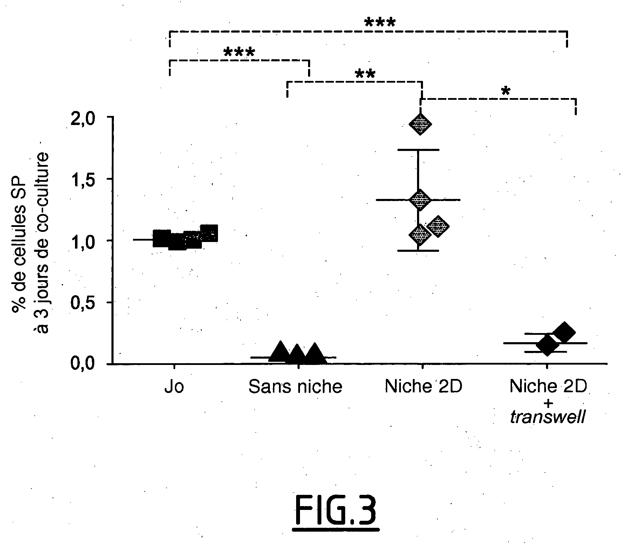

**Figure 3****. Evaluation du maintien/acquisition de la fonctionnalité SP de cellules cultivées sur un support de culture selon l'invention**
   Afin de valider la capacité du support de culture selon l'invention à maintenir un pool de cellules hématopoïétiques primitives, le maintien la fonctionnalité SP des cellules médullaires Lin- a été évalué après 3 à 7 jours de culture. Dans ce but, des cellules médullaires Lin⁻ (2.10⁵) ont été cultivées en présence ou en l'absence de supports de culture cellularisés en 2D (n= 3 à 4). En présence d'un support de culture cellularisé avec des CSM et/ou des CSM différenciées en ostéoblastes, le pourcentage de cellules SP (1.46+/-0.33) est maintenu par rapport au premier jour (avant culture : 1.02+/-0.03, NS), voire augmenté en comparaison à la condition sans support de culture cellularisé (0.05+/- 0.01, p<0.01) et à la condition en transwell (0.17+/-0.05, p<0.05).

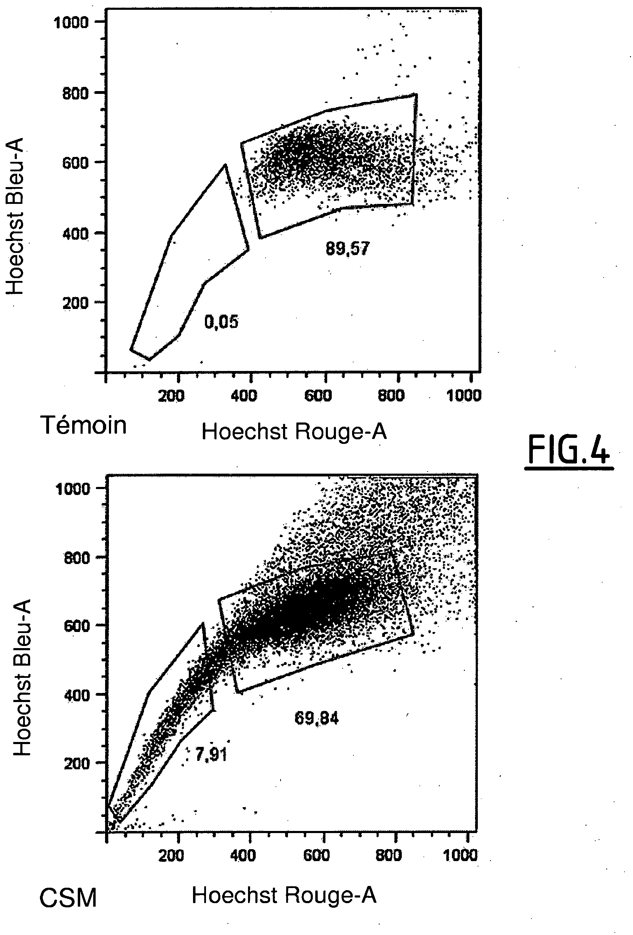

**Figure 4****. Effet des CSM sur l'acquisition d'une fonctionnalité SP**
   Des cellules médullaires Lin⁻ ont été cultivées en présence ou en l'absence de supports de culture en 2D cellularisés avec des CSM.

### EXEMPLES

### Exemple 1: Préparation des différents types cellulaires constituant la niche

### Isolement et culture des Cellules Souches Mésenchymateuses (CSM)

Les cellules mononucléées de moelle osseuse sont isolées à partir de fragments d'os spongieux issus de résidus opératoires de patients opérés pour prothèse totale de hanche et ensemencées dans des flasques de 75 cm² à la concentration de 100.000 cellules/cm² dans un milieu à base de MEMα (ATGC/biological industries) contenant 10% de sérum de veau foetal (SVF, Hyclone) et 1% de ciprofloxacine (Bayer). Les flacons de culture sont incubés à 37 °C dans une atmosphère contenant 5% de CO₂ et 20% d'O₂. Le milieu de culture est renouvelé au bout de trois jours de culture la première semaine puis une fois par semaine jusqu'à quasi-confluence (80% à 90%). Lorsque les cellules ont atteint la confluence, elles sont décollées du support plastique par action enzymatique de la trypsine (trypsine 1 : 250, Sigma) pendant 5 min à 37 °C, numérées (bleu trypan) puis congelées dans une solution cryoprotectrice contenant 5% d'albumine humaine et 10% de DMSO. En vue de leur utilisation, les CSM sont ensuite décongelées au bain marie à 37°C puis reprises et lavées dans un milieu composé de MEMα, 1% ciprofloxacine et 20% d'albumine. Après numération et évaluation de la viabilité par coloration au bleu trypan, les CSM sont ensuite ensemencées dans différentes conditions expérimentales décrites ci-dessous dans un milieu de culture à base dé MEMα, 10% SVF, 1% ciprofloxacine. Le milieu de culture est renouvelé une fois par semaine jusqu'à ce que les cellules atteignent 80% de confluence. Elles sont ensuite trypsinées et réensemencées pour un second passage.

### Différenciation ostéoblastique et adipocytaire des CSM

- *Différenciation ostéoblastique :* Les différenciations ostéoblastiques sont induites à partir de CSM menées à confluence en milieu MEMα, 10% SVF, 1% ciprofloxacine enrichi avec 0,1 µM de dexamethasone, 0,05 mM d'acide L-ascorbique 2 phosphate et 10 mM de β-glycerophosphate (Sigma). Les milieux d'induction sont renouvelés deux fois par semaine pendant trois semaines. La qualité de la différenciation est évaluée au microscope à contraste de phase par appréciation du niveau de minéralisation, par immunohistochimie par mise en évidence de l'activité phosphatase alcaline (PAL) grâce à une réaction chimique au naphtol AS-BIphosphate (Sigma), et par immunofluorescence indirecte (IFI) avec mise en évidence de l'ostéocalcine, de l'ostéopontine et de la PAL. Les réactions d'IFI sont réalisées après fixation au paraformaldéhyde à 4% (Sigma) suivie d'une saturation/perméabilisation par du PBS enrichi par 3% d'albumine bovine et 0,1% de triton X100 (prolabo). Les anticorps anti-ostéocalcine, anti-ostéopontine et anti-PAL sont incubés dans une solution de PBS enrichi par 1% d'albumine bovine et 0,05% de tween 20 (Biorad), puis les marquages sont révélés par un anticorps ciblant les Ig murines couplé à la phycoérythrine (goat antimouse-PE ; Caltag). Enfin, une contre coloration au Hoechst permettant de visualiser les noyaux est réalisée (hoeschst 33258, Molecular Probes).
- *Différenciation adipocytaire :* Les différenciations adipocytaires sont induites à partir de CSM sub-confluentes par trois cycles de traitement avec 1 µM de dexamethasone, 0,5 mM de 3-isobutyl-1-methylxanthine (IBMX), 0,2 mM d'indomethacine et 0,01 mg/ml d'insuline (Sigma). Chaque cycle de traitement dure trois jours, le dernier cycle se terminant par trois jours de maintenance dans un milieu spécifique (10% SVF supplémenté avec 0,01 mg/ml d'insuline). La nature adipocytaire de la culture est affirmée par l'examen microscopique à contraste de phase montrant la présence de vacuoles lipidiques, et par une coloration immunohistochimique à l'oil red O fixant les vacuoles lipidiques.

### Différenciation ostéoclastique à partir des progéniteurs hématopoïétiques

Les cellules mononucléées de moelle osseuse humaine obtenues à partir de fragments d'os spongieux issus de résidus opératoires de patients opérés pour prothèse totale de hanche sont séparées par gradient de densité sur coussin de Ficoll puis mises en culture pendant une nuit, dans une étuve à 37°C, en atmosphère humide contenant 5% de CO₂. Le lendemain, une déplétion Lin- est effectuée sur colonne d'affinité afin d'éliminer les cellules différenciées exprimant les antigènes de lignée suivants : CD2, CD3, CD11b, CD14, CD15, CD16, CD19, CD56, CD123, GPA. Les cellules Lin⁻ ainsi obtenues (environ 10⁷ cellules) sont mises en culture sur des biomatériaux HA/TCP dans un milieu contenant un cocktail de cytokines (SCF, Flt3 et TPO à 10 ng/ml). Les cellules Lin⁻ (2.10⁶ cellules Lin⁻) sont déposées sur les biomatériaux 2D ou 3D. Après 5 jours de culture, le milieu de culture est remplacé par un milieu de différenciation myéloïde contenant les cytokines suivantes : SCF, Flt3, TPO, IL-6 et GM-CSF à 10 ng/ml. Après 15 jours de culture, les cellules sont incubées dans un milieu d'induction ostéoclastique contenant du M-CSF, RANK-L et IL-6 à 20 ng/ml.

La qualité de la différenciation est contrôlée sur un puits ne contenant pas de biomatériau. Le phénotype ostéoclastique est déterminé par coloration de May Grumwald Giemsa (MGG) permettant de mettre en évidence l'aspect multinucléé, résultant de la fusion des cellules ostéoclastiques, et par la mise en évidence de l'activité phosphatase acide tartrate résistante (TRAP).

### Obtention des cellules endothéliales

Les cellules endothéliales peuvent être obtenues à partir de deux sources :
1) des progéniteurs endothéliaux circulant du sang (PEC) de sang périphérique. Pour cela, les colonies dérivées des PEC sont générées à partir des cellules mononucléées obtenues à partir de 20 mL de sang. Chaque colonie produit environ 10⁶ cellules après 2 passages. Ainsi, après 3 ou 4 passages, on peut obtenir jusqu'à 10⁸ cellules endothéliales fonctionnelles
2) des cellules mononuclées de la moelle osseuse. La première étape consiste en une déplétion immunomagnétique des cellules lineage positives, permettant d'éliminer la population majoritaire des cellules hématopoïétiques engagées. Sur la population Lin-, un tri CD144/KDR est alors réalisé, et les cellules triées sont cultivées en milieu endothélial. Les cellules endothéliales médullaires isolées prolifèrent alors rapidement dans ce milieu.

### Isolement des Cellules Souches Hématopoïétiques (CSH) / Progéniteurs Hématopoïétiques (PH)

**Lin⁻ :** Les cellules hématopoïétiques mononucléées médullaires ou sanguines sont obtenues après centrifugation sur un gradient de densité de Ficoll Hypaque. Puis les cellules Lin- (lineage negatives) sont triées après marquage avec un cocktail d'anticorps ciblés contre différents antigènes de différenciation (Lineage cell depletion kit, Miltenyi Biotec). Après un lavage en milieu tampon, le tri est réalisé par déplétion sur un automate (Automacs, Miltenyi Biotech).
**SP :** Les cellules Lin- sont incubées en présence de Hoechst-33342 (5 µg/ml), un intercalant de l'ADN, à raison de 10⁶ cellules/ml dans du DMEM contenant 2% de SVF. Les cellules sont ensuite incubées pendant 90 min à 37°C ; le reste de la manipulation s'effectue à froid pour éviter l'efflux passif du Ho par les cellules n'exprimant pas les pompes. Les cellules sont ensuite centrifugées 15 min, à 4°C et à 1500 rpm et le culot cellulaire est re-suspendu dans du HBSS sans Ca2+, ni Mg2+, à froid, à une concentration de 2 à 4.10⁶ cellules/ml.
**ALDH^{fortes}**: La technologie ALDEFLUOR utilise le substrat Bodipy™-AminoAcétAldéhyde Diéthyl Acétal (BAAA-DA) de l'enzyme ALDH-A1. Ce substrat est dissous dans du DMSO et exposé à l'action de l'HCL afin d'être convertit en BAAA, substrat fluorescent de l'enzyme. Les cellules sont incubées en présence de BAAA (1,5 µM) à 37°C qui diffuse à travers les membranes plasmiques des cellules viables. L'enzyme ALDH convertit ce substrat en un produit fluorescent (BAA) qui est alors retenu à l'intérieur des cellules en raison de sa charge négative et de la polarité des membranes cellulaires. Le tampon d'incubation ALDEFLUOR contient un inhibiteur des pompes d'efflux MDR. En conséquence, les cellules qui expriment un haut niveau d'ALDH sont celles qui retiennent le BAA au sein de leur cytoplasme, mais aussi celles qui présentent un haut niveau de fluorescence. Cette fluorescence peut être mesurée dans le canal FL1 (fluorescence FITC) en cytométrie de flux.
**CD34⁺** : Les cellules CD34⁺ sont purifiées à partir de cellules mononucléées issues d'un échantillon de moelle allogénique. Les cellules mononucléées sont séparées selon un gradient de Ficoll et le culot est repris en tampon PBS enrichi par 2% d'albumine humaine (Vialebex, LFB) et 0,5% d'immunoglobulines polyvalentes humaines (Tégéline, LFB) puis incubé 5 minutes de façon à saturer les récepteurs non spécifiques aux Immunoglobulines (Ig) présents à la surface des cellules CD34⁺. Les cellules sont ensuite incubées en présence d'un anticorps anti-CD34 couplé à des billes magnétiques (20µl/10⁸ cellules ; Clinimacs, Miltenyi Biotech) pendant 30 minutes puis lavées en PBS-2% albumine (PA) avant d'être déposées sur colonne immunomagnétique (Automacs, Miltenyi Biotech). Les cellules peuvent être ensuite congelées en albumine 5%, dimethylsulfoxide (DMSO, Sigma) 10%. La qualité du tri est contrôlée par cytométrie en flux de façon à évaluer la pureté cellulaire en CD34⁺ dans la suspension cellulaire finale.

### Exemple 2 : Choix des biomatériaux

La modélisation de la niche nécessite la mise en commun des différents stromas sur un même support ostéoconducteur en 2D ou en 3D. Ces biomatériaux (BM) peuvent être en particulier constitués d'hydroxyapatite (HA) et de phosphate tricalcique (TCP) en différentes proportions et présenter des porosités variables. Parmi ceux-ci, citons les B2D et B3D (BD Biocoat™ Osteologic™ Bone Cell Culture System) et le Calciresorb 35® (Ceraver) qui est une céramique bi-phasée composée de 65% HA et 35% βTCP. Les résultats présentés dans l'Exemple 5 ont été obtenus avec le Calciresorb 35® (Ceraver).

### Exemple 3 : Choix de la matrice extracellulaire

Des glycosaminoglycanes (GAG) naturels ou leurs mimétiques (qui présentent l'avantage de ne pas être dégradés par des glycanases) peuvent être associés aux biomatériaux. Les GAG mimétiques préférentiellement utilisés sont commercialisés par la société OTR3 (pour Organ, Tissue, Regeneration, Repair and Replacement) sous l'appellation de RGTAs pour « ReGeneraTing Agents » et sont idéalement utilisés à une concentration de 10 à 50 mg/ml. Les résultats présentés dans l'Exemple 5 ont été obtenus avec le OTR4131.

### Exemple 4 : Modélisation de la niche hématopoïétique en 2D ou 3D

La modélisation de la niche nécessitant la mise en commun des différents stromas dans un même puits, les CSM, les ostéoclastes et les cellules endothéliales sont ensemencées idéalement sur un même BM ou sur des BM différents 2D ou 3D (un par type cellulaire) (Figure 1). Les résultats présentés dans l'Exemple 5 ont été obtenus par ensemencement de différents BM 3D de type Calciresorb 35®. Les CSM sont induites vers les différenciations ostéoblastiques et adipocytaires. On obtient ainsi des BM hybrides cellularisés par les différents types de cellules stromales. Ces derniers sont alors rassemblés dans un puits unique contenant l'ensemble des BM hybrides dans lequel sont ensuite ajoutés à concentrations variables des CSH/PH afin de réaliser des co-cultures. Les résultats présentés dans l'Exemple 5 ont été obtenus après ajout de cellules Lin⁻ à la concentration de 1.10⁵ cellules/ml/puits. Une alternative à ce protocole consiste à réaliser des cultures de chaque type cellulaire différencié sous forme de pelotes (en les empêchant d'adhérer au support lors de leur différenciation). Les différents types cellulaires sont ensuite mixés et ensemencés sur un seul et même biomatériau (De Barros et al., PLoS One. 2010;5(2), page 11, 3ème paragraphe).

Dans le détail, dans le cas de la préparation des différents types de cellules stromales sur des BM indépendants, les BM sont ensemencés individuellement dans des microtubes à la concentration de 5.10⁴ à 2.10⁵ cellules par ml et par BM. Après 3 heures d'incubation à 37°C en atmosphère enrichie de 5% de CO₂, les BM cellularisés sont transférés dans une microplaque (quatre puits) dans un milieu MEMα, 10% SVF, 1% ciprofloxacine afin de réaliser les inductions ostéoblastiques et adipocytaires 2 à 5 jours plus tard. La période d'induction ostéoblastique est limitée à quatorze jours car les BM induisent par eux même une auto-induction des CSM vers la lignée ostéoblastique. Plusieurs BM « témoins » (un par type cellulaire) sont analysés en immunohistochimie par réaction au naphtol AS-BIposphate et par coloration à l'oil red O de façon à confirmer la positivité des différenciations ostéoblastiques et adipocytaires.

Les CSH/PH sont décongelées le jour de la mise en co-culture et reprises dans du milieu SYNH (95L01 HSA, AbCys) contenant 1% de ciprofloxacine, enrichi en facteurs de croissance hématopoïétiques (FCH, 10 ng/mL) : thrombopoïétine (Tpo, Peprotech), stem cell factor (SCF, Peprotech) et Flt3-ligand (RetD System). La qualité des échantillons décongelés est évaluée par cytométrie de flux (expression du CD34) et par cultures en milieu semi-solide. Les cellules décongelées sont ensuite ensemencées (5.10⁴ à 2.10⁵ cellules par puits) en présence des différents BM hybrides pour former une niche puis cultivées pendant 1 à 2 semaines dans le milieu SYNH précédemment défini.

Ces cultures sont réalisées soit en concentration relative en oxygène de 20%, soit en condition hypoxique plus proche de la situation physiologique dans la moelle osseuse, c'est-à-dire à des concentrations entre 1 et 5%. Les résultats présentés dans l'Exemple 5 ont été obtenus dans des conditions de concentration relative en oxygène de 3%.

### Exemple 5 : Caractérisation biologique de la niche

### Caractérisation des différents types cellulaires de la niche

L'évaluation de la différenciation ostéoblastique, adipocytaire et ostéoclastique par des tests d'induction spécifique est réalisée à l'aide de coloration, tel que décrit dans l'Exemple 1.

### Evaluation fonctionnelle de la niche

### Evaluation des facteurs secrétés par la niche 3D

Les principales molécules impliquées dans la régulation de l'hématopoièse et produites au sein des niches ainsi modélisées ont été quantifiées par un test ELISA après 7 jours de co-culture en présence de CSH/PH CD34⁺. Les résultats sont exprimés en pg/ml par 10⁵ cellules stromales (CSM, ostéoblastes, ostéoclastes et adipocytes) et 5.10⁴ cellules CD34⁺ initialement ensemencées. Le Tableau 1 ci-dessous montre une production importante de différents facteurs ; les ostéoblastes et les CSM étant les cellules majoritairement productrices. Ces résultats témoignent de la capacité fonctionnelle des cellules constituant les niches ainsi modélisées.

**Tableau 1 : Evaluation des facteurs secrétés par une niche cellularisée**

| **Facteur** | **Concentration à J7 (pg/ml)** |
|---|---|
| Ostéopontine | 17000 ± 5000 |
| Ostéoprotégérine | 9 ± 7 |
| Flt3-L | 21000 ± 8000 |
| Interleukine 8 | 4500 ± 3500 |
| Interleukine 6 | 5000 ± 100 |
| Stem Cell Factor | 5900 ± 100 |
| Angiopoïétine 1 | 800 ± 40 |
| Hepatocyte Growth factor | 1700 ± 50 |
| SDF1 | 380 ± 80 |

### Evaluation du pouvoir stromacytaire des niches 2D et 3D modélisées

Les niches modélisées en 2D (ostéoblastes, adipocytes, CSM et ostéoclastes) favorisent l'expansion des cellules CD34⁺ à partir d'une population de cellules Lin⁻ de sang périphérique de sujets sains non mobilisés (richesse en cellules CD34⁺ équivalente à 30%). Les résultats montrent qu'après 7 jours de co-culture, la population CD34⁺ représente environ 96,25% +/- 1,71% (n=4) des cellules viables produites (Figure 2).

Le pourcentage de cellules CD34⁺ est significativement plus élevé que celui observé en condition dite « sans niche », c'est-à-dire en présence de biomatériaux non colonisés par les cellules stromales (97% +/- 2% vs 85% +/- 8% ; p<0.05). Bien que le taux de prolifération des cellules totales soit légèrement plus faible dans les conditions nichées (7.75% +/- 3.5%) par rapport aux conditions sans niches (13.67% +/- 3.06% ; non significatif), le taux d'amplification du nombre de cellules CD34^{Fort} au sein des niches est environ 3,5 fois plus élevé qu'au jour 0. Au contraire, lorsque les cellules CD34 sont cultivées en conditions «sans niche », le nombre de cellules CD34^{Fort} est inférieur au chiffre initial. Les niches ainsi modélisées permettent l'amplification des cellules CD34^{Fort} au détriment des cellules CD34^{Faible}, ce qui suggère que la prise en compte de la niche pourrait avoir un intérêt dans l'optimisation des protocoles de culture des cellules CD34^{Fort}, permettant de favoriser la prolifération au dépend de la différenciation.

Par ailleurs, lorsque les cellules CD34⁺ sont séparées de la niche 2D par un système de transwell, les résultats en termes de maintien du nombre de cellules CD34⁺ sont inférieurs à la condition reproduisant une niche complète, ce qui suggère l'importance des contacts cellulaires et des facteurs sécrétés majoritairement par les cellules stromales dans ce processus.

### Evaluation du maintien/acquisition de la fonctionnalité SP par les niches modélisées

Afin de valider la capacité des niches hématopoïétiques à maintenir un pool de cellules hématopoïétiques primitives, nous avons également évalué leur capacité à maintenir la fonctionnalité SP des cellules médullaires Lin⁻ après 3 à 7 jours de culture. La fonctionnalité SP est définie par la capacité des cellules souches et en particulier des CSH d'effluer le colorant fluorescent Hoechst-33342 ou d'autres colorants et drogues. Cette fonctionnalité peut être évaluée par cytométrie en flux par quantification de la fluorescence résiduelle des cellules sous la forme d'un cytogramme caractéristique, les cellules ayant la plus forte capacité d'efflux étant considérées comme les plus primitives. Dans ce but, nous avons cultivé des cellules médullaires Lin⁻ (2.10⁵) en présence ou en l'absence de niches cellularisées en 2D (n= 3 à 4).

La Figure 3 montre qu'en présence de niches cellularisées (ostéoblastes, adipocytes, ostéoclastes, CSM), les cellules médullaires Lin⁻ conservent leur fonctionnalité SP, comparativement aux co-cultures sur des biomatériaux non cellularisés (condition sans niche) ou en système transwell (condition Boyden). Ainsi, en présence d'une niche cellularisée, le pourcentage de cellules SP (1.46+/-0.33) est maintenu par rapport au premier jour (avant culture : 1.02+/-0.03, NS), voire augmenté en comparaison à la condition sans niche (0.05+/-0.01, p<0.01) et à la condition en transwell (0.17+/-0.05, p<0.05). Ainsi, en termes de nombre absolu de cellules SP, la culture sur une niche 2D permet de maintenir le pool de cellules SP en coculture pendant trois jours (environ 2920, en présence de niche), ce qui n'est pas le cas en absence de niche ou en condition transwell (environ 100, en absence de niche). Ces résultats suggèrent que les interactions cellulaires entre les cellules stromales et les cellules hématopoïétiques sont nécessaires au maintien de la fonctionnalité SP.

Contrairement aux CSH/PH de moelle osseuse, les cellules du sang périphérique n'expriment pas la fonctionnalité SP. Quand ces dernières sont ensemencées pendant 3-5 jours sur une niche modélisée, un certain pourcentage (0.5 à 2%) d'entre elles acquière la fonctionnalité SP. Des expériences réalisées en utilisant les différents constituants cellulaires séparément montrent que les CSM semblent avoir un rôle prépondérant dans cette acquisition (Figure 4).

## Revendications

1. Support de culture de cellules souches hématopoïétiques (CSH) et/ou progéniteurs hématopoïétiques (PH), comprenant :
a. un biomatériau calcique ;
b. des ostéoclastes ;
c. des cellules endothéliales ; et
d. des cellules souches mésenchymateuses (CSM) et/ou des ostéoblastes et/ou adipocytes.

2. Support de culture selon la revendication 1, dans lequel ledit biomatériau calcique comprend de l'hydroxyapatite (HA) et du phosphate tricalcique (TCP).

3. Support de culture selon la revendication 1 ou 2, dans lequel ledit biomatériau calcique est bidimensionnel ou tridimensionnel.

4. Support de culture selon l'une quelconque des revendications 1 à 3, dans lequel ledit biomatériau calcique est sélectionné dans le groupe constitué des biomatériaux B2D et B3D (BD Biocoat™ Osteologic™ Bone Cell Culture System) et du Calciresorb 35® (Ceraver, France).

5. Support de culture selon l'une quelconque des revendications 1 à 4, qui comprend en outre un ou des composants de la matrice extracellulaire (MEC).

6. Support de culture selon l'une quelconque des revendications 1 à 5, qui comprend en outre une colle biologique.

7. Méthode de préparation d'un support de culture tel que défini dans l'une quelconque des revendications 1 à 6, comprenant les étapes consistant à greffer au moins un biomatériau calcique avec des ostéoclastes, des cellules endothéliales et des cellules souches mésenchymateuses (CSM) et/ou des ostéoblastes et/ou adipocytes, et optionnellement un ou des composants de la MEC.

8. Méthode de culture *in vitro* de cellules souches hématopoïétiques (CSH) et/ou progéniteurs hématopoïétiques (PH), comprenant les étapes consistant à :
a. ensemencer au moins un support de culture tel que défini dans l'une quelconque des revendications 1 à 5, avec des cellules souches hématopoïétiques (CSH) et/ou progéniteurs hématopoïétiques (PH) ; et
b. cultiver lesdites cellules souches hématopoïétiques (CSH) et/ou progéniteurs hématopoïétiques.

9. Utilisation d'un support de culture tel que défini dans l'une quelconque des revendications 1 à 6 pour étudier les mécanismes cellulaires impliqués dans l'hématopoïèse et/ou la différenciation des cellules souches hématopoïétiques (CSH) et/ou progéniteurs hématopoïétiques (PH).

10. Utilisation d'un support de culture tel que défini dans l'une quelconque des revendications 1 à 6 pour évaluer le potentiel de nichage d'un greffon hématopoïétique.

11. Utilisation d'un support de culture tel que défini dans l'une quelconque des revendications 1 à 6 pour étudier l'efficacité et/ou la toxicité d'un candidat médicament.

12. Kit comprenant :
a. un biomatériau calcique ;
b. des ostéoclastes, ou un ensemble de cytokines et/ou facteurs de croissance permettant la différenciation de cellules souches hématopoïétiques (CSH) et/ou progéniteurs hématopoïétiques (PH) en ostéoclastes;
c. des cellules endothéliales, ou un ensemble de cytokines et/ou facteurs de croissance permettant la différenciation de progéniteurs endothéliaux circulants ou de cellules mononuclées de la moelle osseuse en cellules endothéliale ;
d. des cellules souches mésenchymateuses (CSM) et/ou des ostéoblastes et/ou des adipocytes et éventuellement un ensemble de cytokines et/ou facteurs de croissance permettant la différenciation des CSM en ostéoblastes/adipocytes ; et/ou des ostéoblastes et/ou adipocytes ; et
e. éventuellement un ou des composants de la matrice extracellulaire (MEC).

13. Support de culture selon la revendication 2, dans lequel la proportion en HA du biomatériau est comprise entre 55% et 75% et/ou la proportion en TCP du biomatériau est comprise entre 25% et 45%.

14. Support de culture selon l'une quelconque des revendications 1 à 6, dans lequel ledit biomatériau calcique est tridimensionnel.

15. Méthode de préparation d'un support de culture selon la revendication 7, dans laquelle la préparation du support de culture est réalisée dans des conditions telles que la teneur en oxygène est comprise entre 1 et 5%.

## Patentansprüche

1. Kulturmedium für hämatopoetische Stammzellen (CSH) und/oder hämatopoetische Vorläuferzellen (PH), aufweisend:
a. ein Calcium-Biomaterial;
b. Osteoklasten;
c. Endothelzellen; und
d. mesenchymatische Stammzellen (CSM) und/oder Osteoblasten und/oder Fettzellen.

2. Kulturmedium gemäß Anspruch 1, wobei das genannte Calcium-Biomaterial Hydroxylapatit (HA) und Tricalciumphosphat (TCP) aufweist.

3. Kulturmedium gemäß Anspruch 1 oder 2, wobei das genannte Calcium-Biomaterial zweidimensional oder dreidimensional ist.

4. Kulturmedium gemäß irgendeinem der Ansprüche 1 bis 3, wobei das genannte Calcium-Biomaterial ausgewählt ist aus der Gruppe bestehend aus den Biomaterialien B2D und B3D (BD Biocoat™ Osteologic™ Bone Cell Culture System) und Calciresorb 35® (Ceraver, Frankreich).

5. Kulturmedium gemäß irgendeinem der Ansprüche 1 bis 4, ferner aufweisend einen oder mehrere Bestandteile der extrazellulären Matrix (MEC).

6. Kulturmedium gemäß irgendeinem der Ansprüche 1 bis 5, ferner aufweisend einen biologischen Klebstoff.

7. Verfahren zum Herstellen eines Kulturmediums gemäß irgendeinem der Ansprüche 1 bis 6, wobei das Verfahren die Schritte aufweist Übertragen von Osteoklasten, Endothelzellen und mesenchymatischen Stammzellen (CSM) und/oder Osteoblasten und/oder Fettzellen und optional einem oder mehreren Bestandteilen der MEC auf mindestens ein Calcium-Biomaterial.

8. *In vitro*-Zellkulturverfahren von hämatopoetischen Stammzellen (CSH) und/oder hämatopoetischen Vorläuferzellen (PH), wobei das Verfahren die Schritte aufweist:
a. Beimpfen mindestens eines Kulturmediums gemäß irgendeinem der Ansprüche 1 bis 5 mit hämatopoetischen Stammzellen (CSH) und/oder hämatopoetischen Vorläuferzellen (PH); und
b. Kultivieren der genannten hämatopoetischen Stammzellen (CSH) und/oder der hämatopoetischen Vorläuferzellen (PH).

9. Verwenden eines Kulturmediums gemäß irgendeinem der Ansprüche 1 bis 6 zum Untersuchen der Zellmechanismen, die an der Hämatopoese und/oder der Differenzierung von hämatopoetischen Stammzellen (CSH) und/oder von hämatopoetischen Vorläuferzellen (PH) beteiligt sind.

10. Verwenden eines Kulturmediums gemäß irgendeinem der Ansprüche 1 bis 6 zum Evaluieren des Anwachspotentials eines hämatopoetischen Transplantats.

11. Verwenden eines Kulturmediums gemäß irgendeinem der Ansprüche 1 bis 6 zum Untersuchen der Wirksamkeit und/oder der Toxizität eines Wirkstoffkandidaten.

12. Kit, aufweisend:
a. ein Calcium-Biomaterial;
b. Osteoklasten oder eine Zusammensetzung aus Cytokinen und/oder Wachstumsfaktoren, die die Differenzierung von hämatopoetischen Stammzellen (CSH) und/oder von hämatopoetischen Vorläuferzellen (PH) zu Osteoklasten erlauben;
c. Endothelzellen oder eine Zusammensetzung aus Cytokinen und/oder Wachstumsfaktoren, die die Differenzierung von zirkulierenden Endothel-Vorläuferzellen oder von mononukleären Zellen des Knochenmarks zu Endothelzellen erlauben;
d. mesenchymatische Stammzellen (CSM) und/oder Osteoblasten und/oder Fettzellen und gegebenenfalls eine Zusammensetzung aus Cytokinen und/oder Wachstumsfaktoren, die die Differenzierung von CSM zu Osteoblasten/Fettzellen erlauben; und/oder Osteoblasten und/oder Fettzellen; und
e. gegebenenfalls einen oder mehrere Bestandteile der extrazellulären Matrix (MEC).

13. Kulturmedium gemäß Anspruch 2, wobei der Anteil des HA an dem Biomaterial zwischen 55% und 75% beträgt und/oder der Anteil des TCP an dem Biomaterial zwischen 25% und 45% beträgt.

14. Kulturmedium gemäß irgendeinem der Ansprüche 1 bis 6, wobei das genannte Calcium-Biomaterial dreidimensional ist.

15. Verfahren zum Herstellen eines Kulturmediums gemäß Anspruch 7, wobei das Herstellen des Kulturmediums unter Bedingungen stattfindet, in denen der Sauerstoffgehalt zwischen 1 und 5% beträgt.

## Claims

1. A culture support of hematopoietic stem cells (HSCs) and/or hematopoietic progenitors (HPs), comprising:
a. a calcium biomaterial;
b. osteoclasts;
c. endothelial cells; and
d. mesenchymatous stem cells (MSCs) and/or osteoblasts and/or adipocytes.

2. The culture support according to claim 1, wherein said calcium biomaterial comprises hydroxyapatite (HA) and tricalcium phosphate (TCP).

3. The culture support according to claim 1 or 2, wherein said calcium biomaterial is two-dimensional or three-dimensional.

4. The culture support according to any of claims 1 to 3, wherein said calcium biomaterial is selected from the group consisting of the biomaterials B2D and B3D (BD Biocoat^{™} Osteologic^{™} Bone Cell Culture System) and Calciresorb 35® (Ceraver, France).

5. The culture support according to any of claims 1 to 4, which further comprises one or more component(s) of the extracellular matrix (ECM).

6. The culture support according to any of claims 1 to 5, which further comprises a biological glue.

7. A method for preparing a culture support as defined in any of claims 1 to 6, comprising the steps consisting of grafting at least one calcium biomaterial with osteoclasts, endothelial cells and mesenchymatous stem cells (MSCs) and/or osteoblasts and/or adipocytes, and optionally one or more component(s) of the ECM.

8. An *in vitro* method for culturing hematopoietic stem cells (HSCs) and/or hematopoietic progenitors (HPs), comprising the steps consisting of:
a. seeding at least one culture support as defined in any of claims 1 to 5, with hematopoietic stem cells (HSCs) and/or hematopoietic progenitors (HPs); and
b. culturing said hematopoietic stem cells (HSCs) and/or hematopoietic progenitors.

9. Use of a culture support as defined in any of claims 1 to 6 for studying the cellular mechanism involved in hematopoiesis and/or differentiation of hematopoietic stem cells (HSCs) and/or hematopoietic progenitors (HPs).

10. Use of a culture support as defined in any of claims 1 to 6 for evaluating the nesting potential of a hematopoietic graft.

11. Use of a culture support as defined in any of claims 1 to 6 for studying the efficiency and/or the toxicity of a medicament candidate.

12. A kit comprising:
a. a calcium biomaterial;
b. osteoclasts, or a set of cytokines and/or growth factors allowing differentiation of hematopoietic stem cells (HSCs) and/or hematopoietic progenitors (HPs) into osteoclasts;
c. endothelial cells, or a set of cytokines and/or growth factors allowing differentiation of circulating endothelial progenitors or bone marrow mononuclear cells into endothelial cells;
d. mesenchymatous stem cells (MSCs) and/or osteoblasts and/or adipocytes and optionally a set of cytokines and/or growth factors allowing differentiation of MSCs into osteoblasts/adipocytes; and/or osteoblasts and/or adipocytes; and
e. optionally one or more component(s) of the extracellular matrix (ECM).

13. The culture support according to claim 2, wherein the HA proportion of the biomaterial is comprised between 55% and 75% and/or the TCP proportion of the biomaterial is comprised between 25% and 45%.

14. The culture support according to any of claims 1 to 6, wherein said calcium biomaterial is three-dimensional.

15. The method for preparing a culture support according to claim 7, wherein the preparation of the culture support is carried out under conditions such that the oxygen content is comprised between 1 and 5%.
